# EUROPEAN PATENT APPLICATION

(11) **EP 1 260 232 A1**
(43) Date of publication of application: **27.11.2002**
(21) Application number: 01908229.6
(22) Date of filing: 02.03.2001
(51) Int. Cl.: A61K 45/00, A61K 31/4422, A61P 27/02, A61P 25/00, A61P 9/10, A61P 43/00

(54) **REMEDIES FOR MALIGNANT TUMOR-CONCOMITANT NEUROSIS AND AZOOR OR ANALOGOUS DISEASES THEREOF**

(30) Priority: 03.03.2000 JP 2000058339
(71) Applicant: FUJISAWA PHARMACEUTICAL CO., LTD., Osaka-shi Osaka 541-8514 (JP)
(72) Inventor: OHGURO, Hiroshi, Department of Ophthalmology, Hirosaki-shi, Aomori 036-8562 (JP)
(74) Representative: Gille Hrabal Struck Neidlein Prop Roos
(86) International application number: JP0101600
(87) International publication number: WO01064250

(57) **Abstract**

A therapeutic agent for cancer-associated neuropathy and AZOOR or related diseases thereof, which contains, as an active ingredient, a calcium antagonist, particularly a dihydropyridine compound represented by the general formula (I): 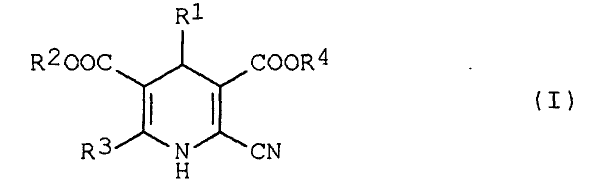 wherein R¹ represents nitrophenyl, and R², R³ and R⁴ each represents a lower alkyl group, or a salt thereof.

## Description

### TECHNICAL FIELD

The present invention relates to a pharmaceutical agent having a therapeutic effect on cancer-associated neuropathy and AZOOR or related diseases thereof.

Particularly, the present invention relates to a therapeutic agent for cancer-associated neuropathy and AZOOR or related diseases thereof, which contains, as an active ingredient, a calcium antagonist such as dihydropyridine-based calcium antagonist, piperazine-based calcium antagonist, phenylalkylamine-based calcium antagonist, benzothiazepin-based calcium antagonist or myosin light chain kinase (MLCK) inhibitorycalciumantagonist. More particularly, the present invention relates to a therapeutic agent for cancer-associated neuropathy and AZOOR or related diseases thereof, which contains, as an active ingredient, a dihydropyridine compound represented by the following general formula (I): 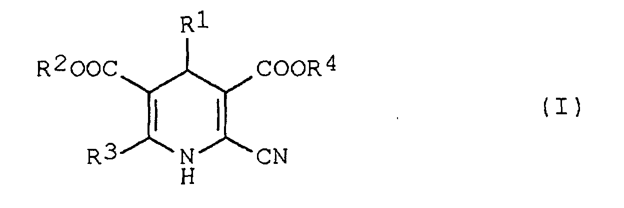 wherein R¹ represents nitrophenyl, and R², R³ and R⁴ each represents a lower alkyl group.

### BACKGROUND ART

It has been known that the calcium antagonist, for example, a dihydropyridine compound represented by the general formula (I), particularly nilvadipine wherein R¹ represents 3-nitrophenyl, R² represents isopropyl and R³ and R⁴ each represents methyl blocks an L type potential-dependent calcium channel activated by depolarization of the cell membrane, thereby to suppress intracellular influx of calcium. From a clinical point of view, the calcium antagonist has widely been used as a hypotensive agent. Furthermore, it has been reported that the dihydropyridine compound (I), particularly nilvadipine exerts an effective therapeutic effect on visual field defects, optic neuropathy, retinopathy and retinal degeneration disease accompanied by normal intraocular pressure glaucoma by enhancing blood flow of the peripheral blood vessel in the ocular tissue such as optic disc, choroid or retina (International Publication WO 97/40834). However, it has never been known what action these calcium antagonists will exert on cancer-associated neuropathy and AZOOR or related diseases thereof.

The present inventors have researched about a pharmacological action of the calcium antagonist including nilvadipine as a therapeutic agent for cancer-associated neuropathy and AZOOR or related diseases thereof, and also studied about the calcium antagonist such as nilvadipine, safety and less side effect of which have already been known, in order to search new pharmaceutical indication in the field of ophthalmology, etc.

### DISCLOSURE OF THE INVENTION

The present inventors have intensively studied about the pharmacological action of the calcium antagonist as the therapeutic agent for cancer-associated neuropathy and AZOOR or related diseases thereof and found that the calcium antagonist exerts an excellent action. Also it has been found that the effect of a dihydropyridine compound (I), particularly nilvadipine, as a therapeutic agent for cancer-associated neuropathy and AZOOR or related diseases thereof can be expected.

That is, the present invention provides a therapeutic agent for cancer-associated neuropathy and AZOOR or related diseases thereof, which contains, as an active ingredient, a calcium antagonist such as dihydropyridine-based calcium antagonist, piperazine-based calcium antagonist, phenylalkylamine-based calcium antagonist, benzothiazepin-based calcium antagonist or amyosin light chain kinase (MLCK) inhibitory calcium antagonist, particularly a dihydropyridine compound represented by the following general formula (I): 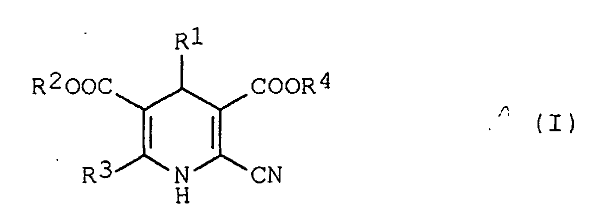 wherein R¹ represents nitrophenyl, and R², R³ and R⁴ each represents a lower alkyl group, or a salt thereof, more particularly isopropyl 6-cyano-5-methoxycarbonyl-2-methyl-4-(3-nitrophenyl)-1,4-di hydropyridine-3-carboxylate ester (generic name: nilvadipine).

More preferably, the present invention provides a therapeutic agent for cancer-associated neuropathy and AZOOR or related diseases thereof, which contains, as an active ingredient, a calcium antagonist represented by the dihydropyridine compound (I) or salt thereof, particularly nilvadipine.

First, target diseases in the present invention will be explained before explaining the respective constituent features of the present invention.

### "Cancer-associated neuropathy"

A part of cancer patients with abnormal central nerve system are generally termed cancer-associated neuropathy, among which cases with a visual system disorder mainly resulting from abnormal retina are termed cancer-associated retinopathy (CAR) . To date, two types of CAR are known: CAR in the narrow sense, which is associated with epithelial cancer (most of cases are lung small cell carcinoma), and melanoma-associated retinopathy (MAR), which is associated with melanoma and shows a clinical profile different from that of CAR. CAR is characterized by retinitis pigmentosa-like progressive retinal degeneration. In most CAR cases, retinopathy is found prior to detection of the primary cancer lesion in clinical practice. On the other hand, MAR shows a relatively slow clinical course and is characterized by negative electroretinogram (ERG) in congenital stationary night blindness. The mechanism of onset of these cancer-associated retinopathies is thought to be generation of retina-specific autoantibodies. In CAR, recoverin, 70 kDa protein and neurofilament are assumed to be autoantigens. In contrast, autoantigens in MAR have not been identified yet, although patient sera would specifically recognize retinal ganglion cells by immunostaining (Nippon Ganka Gakkai Zasshi 101: 283-287, 1997).

### "AZOOR (Acute Zonal Occult Outer Retinopathy)"

AZOOR is a disease firstly published by Grass in 1993, and is predominant in young women. It is characterized by rapid loss of one or more zones of outer retinal function and photopsia with minimal fundoscopic changes, as well as by unilateral or bilateral abnormal eyes recognized by electroretinography. This disease is also characterized by persistent visual field defect during follow-up observation and partial atrophy in the pigment epithelium. Although there is no therapeutic method established for this disease, some reports sate that systemic administration of steroid was effective in some cases.

### " related diseases of AZOOR"

AZOOR-like diseases that show specific fundus findings characterized by enlargement of Mariotte blind spot, including MEWDS (multiple evanescent whitedot syndrome), AIBSES (acute idiopathic blid spot enlargement syndrome), AMN (acute macular neuroretinopathy) and P-POHS (psuedo-presumed ocular histoplasmosis syndrome), may have a common cause with AZOOR. Thus, these diseases are considered as AZOOR related diseases.

The dihydropyridine compound (I) used in the present invention will now be explained.

In the dihydropyridine compound (I), examples of nitrophenyl represented by R¹ include 2-nitrophenyl, 3-nitrophenyl and 4-nitrophenyl, while examples of the lower alkyl group represented by R², R³ and R⁴ include methyl, ethyl, propyl, isopropyl, butyl, tertiary butyl, pentyl and hexyl.

Preferred salt in the dihydropyridine compound (I) is not specifically limited as far as it is a pharmaceutically acceptable salt thereof, and examples thereof include commonly used nontoxic salts, for example, organic acid addition salts such as acetate, trifluoroacetate, maleate, tartrate, methanesulfonate, benzenesulfonate, formate and toluenesulfonate; inorganic acid addition salts such as hydrochloride, hydrobromate, hydroiodate, sulfate, nitrate and phosphate; and salts with acidic amino acids, such as aspartate and glutamate.

The therapeutic agent for cancer-associated neuropathy and AZOOR or related diseases thereof in the present invention is formulated into a dosage form such as oral medication or parenteral medication of a solid, semisolid or liquid by using, as an active ingredient, the calcium antagonist, particularly a dihydropyridine compound (I) or a salt thereof, representatively nilvadipine, and adding an inorganic or organic carrier (which is not specifically limited as far as it is a pharmaceutically acceptable carrier component).

As described above, the drug of the present invention can be provided in any form of an oral medication and a parenteral medication, and also an optimum dosage form can be selected according to the administration route and subjects/animals administered. Examples of the dosage form suited for oral administration include tablets, pills, powders, granules, soft and hard capsules, pellets, sublingual tablets, troches and various solutions, examples of the dosage form suited for parenteral administration include injections, drip infusions, transfusions, suspensions, oil solutions, emulsions and suppositories, and examples of the dosage form suited for topical administration include ophthalmic ointments, eyedrops and sprays.

Using the active ingredient of the present invention, formulation may be conducted in accordance with a conventional procedure, and surfactants, excipients, colorants, perfumes, preservatives, stabilizers, buffers, suspending agents, isotonizing agents and commonly used carriers (which are not specifically limited as far as they are pharmaceutically acceptable carriers) are appropriately used. It is also possible to apply various new dosage forms which are known as the name of DDS.

The dose of the dihydropyridine compound (I) or salt thereof, representatively nilvadipine, is appropriately decided taking into account the type of dosage form, administration method, age and body weight of patients, and type and severity of symptoms. Usually, it is orally administered at a daily dose of about 0.1 to 100 mg, and preferably 1 to 16 mg. An effective single dose is selected within a range from about 0.001 to 1 mg, and preferably from 0.01 to 0.16 mg, per kg of the patient.

The effect of the compound of the present invention will be described to clarify utility of the present invention.

### [Example of Experiment (Therapeutic Effect on Cancer-Associated Neuropathy)]

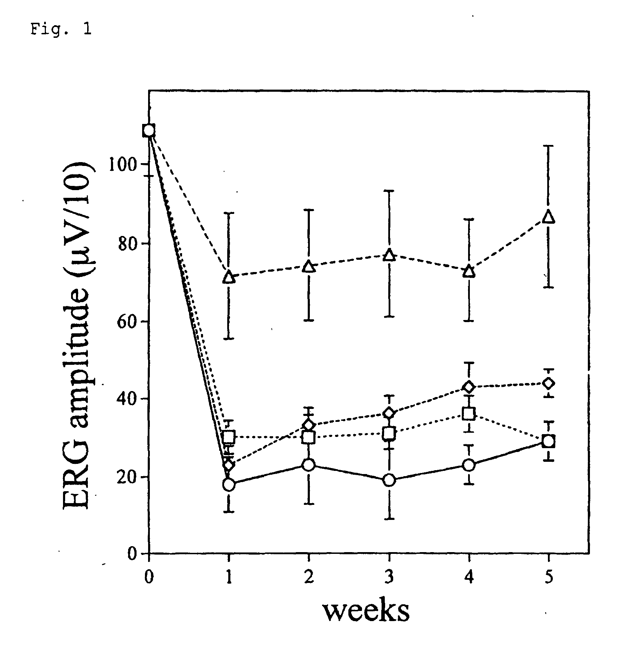

- Test animal:: Lewis rats (8 weeks of age)
Treatment group 5 animals (10 eyes) Control group 5 animals (10 eyes)
- Photoenvironment:: Apoptosis (cell death) was induced under light for 24 hours.
- Test drugs:: Nilvadipine (3.2 mg/kg/day) by peritoneal administration
(dissolving solution) polyethylene glycol 400: ethanol: water = 1:1:2
Predonine (0.6 µg/kg/day) by intramuscular injection
(dissolving solution) PBS (phosphate buffer solution)
Cyclosporine A (10 mg/kg/day) by intramuscular injection
(dissolving solution) olive oil
- Antibodies:: anti-recoverin antibody (5 µl/eye)
anti-hsp70 antibody (5 µl/eye)
- Administration method:: Test drugs were administered for two weeks along with infusion of antibodies into vitreous body of the eye.
Follow-up observation was performed for five weeks after the start of administration. PBS alone was administered to the control group.
- Results:: The results are shown in Fig. 1.

In Fig. 1, the vertical axis represents ERG (electroretinogram) amplitude, and the horizontal axis is the lapse of time in weeks. Δ: nilvadipine group; □: predonine group; ◇: cyclosporin A group; and ○: control group.

These results revealed that nilvadipine is more effective than predonine and cyclosporin A.

### [Example of Clinical Trial (Therapeutic Effect on AZOOR)]

- Case:: Male, 52-year old
- History of the present illness:: The patient became aware of dark and blurred vision in the center of his visual field on September 1, 1999, and visited Ohyachi Kyoritsu Eye Clinic on September 4 in the same year.. His visual acuity was Vd=1.2, Vs=1.2 and showed RAPD(+) in the right eye, although no abnormality was observed in the anterior ocular segment. For ocular fundus, mild turbidity was observed in the vitreous body of the right eye, and the border of the optic disc of the right eye appeared rather unclear. The patient exhibited no symptoms of other diseases, such as cold, before and after the development of the present eye symptom. ERG was subnormal and normal for the right and left eyes, respectively. With respect to the kinetic visual field, enlargement of Mariotte blind spot and reduction of the sensitivity in the central visual field was observed in the right eye. FAG detected mild leakage of fluorescein from the optic disc of the right eye. In view of these observations, optic nerve disorder was suspected and the patient was made to visit a brain surgeon in the neighborhood to receive CT and MRT examinations. However, no abnormality was found. Whole body examination of the patient by a neighboring physician revealed no abnormalities. To treat the eye symptom, the clinic prescribed oral medications such as drug for ameliorating circulation for a while, but no improvement of visual field was observed. Thus, on October 1, 1999, the patient was referred to the Department of ophthalmology at Sapporo Medical University, School of Medicine for close examination.
- Results of the first examination:: Visual acuity was Vd=1.2 and Vs=1.2. Although pupils were round and equal in size, the right eye was RAPD(+) but no oculomotor abnormalities were observed. The right eye had a mild blepharoptosis. Examination of the anterior ocular segment revealed (++) and (-) for cells in the anterior chambers of right and left eyes, respectively, but no abnormalities were observed in the ocular fundus. In the static visual field examination, reduction of the sensitivity in the central visual field was observed.
- Course of disease:: Oral administration of prednisolone (40mg/day) was started on October 1, 1999. The patient became aware of dark and blurred vision also in the center of the visual field of his left eye on October 20, 1999. The dose of prednisolone was reduced to 30 mg/day from October 21 and further to 20 mg/day from October 28, 1999. During this course of treatment, no amelioration of the visual field defect was observed in the right eye (Figs. 2 and 3), but instead, visual field defect in the left eye progressed. The patient was hospitalized on November 9, 1999 for close examination of uveitis and visual field defect. Oral administration of prednisolone could not improve the visual field, and the dose of prednisolone was further reduced to 10 mg/day from November 12, 1999. On November 13, 1999, 0.3 ml of Depo-Medrol was administered to the both eyes by sub-Tenon's injection, and oral administration of nilvadipine was started on November 16, 1999. Two months later, remarkable improvement was observed even in the static visual field examination (Figs. 4 and 5).

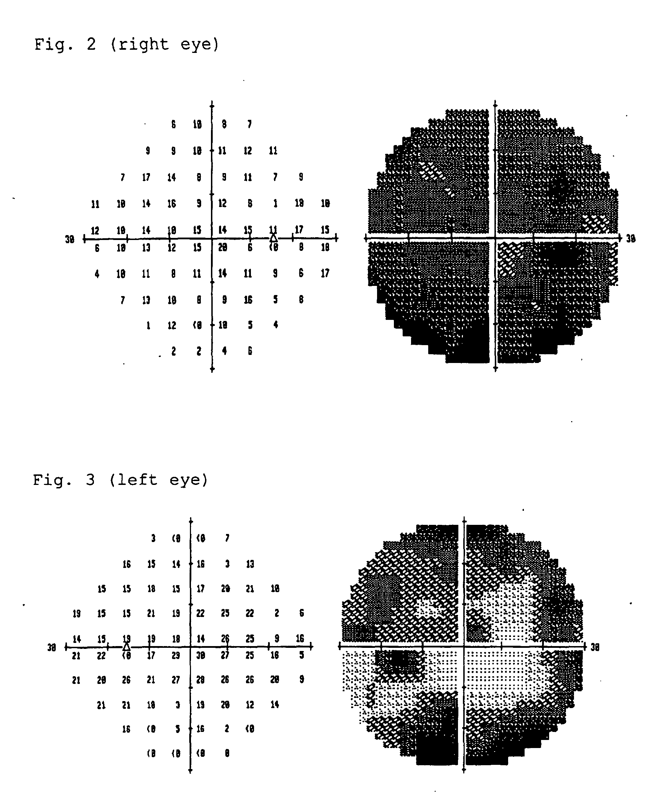

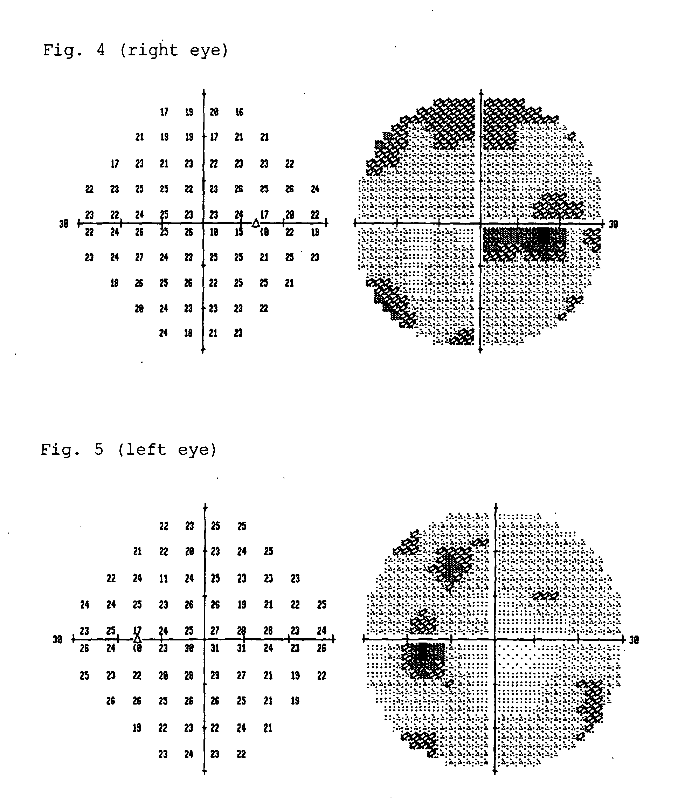

In Figs 2 to 5, the darker shaded areas and the smaller numbers represent more reduction in visual sensitivity. In Figs 2 and 3, marked reduction in visual sensitivity was observed within 30 degrees from the center. By contrast, two months after the administration of nilvadipine, remarkable improvement of visual sensitivity was observed as shown in Figs 4 and 5.

AZOOR is predominantly developed in young women (77%), and has been reported to be characterized by the following features:
1. Rapid loss of one or more zones or wide zone of outer retinal function
2. Trace fundoscopic changes
3. Electroretinographic abnormalities
4. Persistent visual field defect
5. Delayed atrophic changes on the pigment epithelium and narrowing of retinal vessel. Retinal photoreceptor cells and pigment epithelium are considered to be the primary lesion sites of the disease, the pathogenesis of which might be immune-related disorders, invasion of toxic materials into cells and viral infection.

The present case showed the characteristics common to AZOOR, MEWDS, AIBSES, AMN and P-POHS (1. rapid enlargement of Mariotte blind spot without fundus lesions; 2. electroretinographic abnormalities with good eyesight). The patient was diagnosed with AZOOR, since no retinal serum autoantibodies observed in cancer-associated retinopathy and recoverin retinopathy were found by detection of serum autoantibody. This was consistent with the report by Gass *et. al.* In this report, assuming that AZOOR is an autoimmune disorder predominant in women, the authors tested patients' sera by using immunofluorescence on sections of (fixed and unfixed) human and rat retina but failed to detect a retina-specific antibody.

In general, prognosis of AZOOR for visual acuity is said to be good (0.8 or higher for most cases) . However, vision loss has been reported in some cases so far, and therefore long-term follow-up examination is required. Although a few cases have been reported for the effect of the drugs, such as the case whose symptoms were improved by oral administration or sub-Tenon's injection of steroid (60-80 mg), and the case with progress of the extensive visual field defect in the other eye stopped by administration of aciclovir for two weeks, therapeutic effect of these drugs has not been observed inmost cases. In the present case, steroid (40 mg) was orally administered initially, but reduction in visual field sensitivity progressed and the other eye also developed a visual field defect. However, sub-Tenon's injection of steroid and administration of a calcium antagonist exhibited remarkable improvement of visual field sensitivity. Nilvadipine, a calcium antagonist, was developed as a therapeutic agent for hypertension. Recently, as it was found to have an inhibitory effect on apoptosis of cerebral nerve cells, it is now used for treatment of cranial nerve disorder after a stroke. In addition, in the field of ophthalmology, it is suggested to have a neuroprotective effect on normal intraocular pressure glaucoma. In view of these facts, it may be possible for the present case that administration of nilvadipine might have resulted in recovery of visual cell function via a similar mechanism.

Nilvadipine has been launched ten years ago, and has no noteworthy serious side effects. Therefore, it may be possible to use this drug for treatment of the disease in question.

There is a possibility that an agent having a calcium antagonism can be used as a principal agent of the present invention, in addition to the dihydropyridine compound (I) including nilvadipine. Specific examples of these compounds include the following compounds.
Dihydropyridine-based calcium antagonists:
   aranidipine, efonidipine hydrochloride, nicardipine hydrochloride, barnidipine hydrochloride, benidipine hydrochloride, manidipine hydrochloride, cilnidipine, nisoldipine, nitrendipine, nifedipine, felodipine, amlodipine besylate and the like
Piperazine-based calcium antagonists:
   flunarizine hydrochloride, lomerizine hydrochloride and the like;
Phenylalkylamine-based calcium antagonists:
   verapamil hydrochloride and the like
Benzothiazepin-based calcium antagonists:
   diltiazem hydrochloride and the like
Myosin light chain kinase (MLCK) inhibitory calcium antagonists: fasudil hydrochloride hydrate, bepridil hydrochloride and the like

The following examples further illustrate the present invention. The present invention is not limited to the following examples, and various variations made in accordance with the purports described hereinbefore and hereinafter are also included in the technical scope of the present invention.

| Example 1 | |
|---|---|
| Nilvadipine | 100 g |
| Hydroxypropylmethylcellulose | 500 g |

Nilvadipine is dissolved in anhydrous ethanol (5 liter) and hydroxypropylmethylcellulose is added to the resulting solution to prepare a suspension. Then, the organic solvent is removed under reduced pressure to obtain a solid dispersion composition.

| Example 2 | |
|---|---|
| Nilvadipine | 100 g |
| Hydroxypropylmethylcellulose | 500 g |
| Sucrose | 9.4 kg |

Sucrose is added to a suspension of nilvadipine and hydroxypropylmethylcellulose in anhydrous ethanol (5 liter) and the resulting mixture is stirred. Then, the organic solvent is removed under reduced pressure to obtain a solid dispersion composition. This composition is formulated into fine granules in accordance with a conventional procedure.

| Example 3 | |
|---|---|
| Nilvadipine | 100 g |
| Hydroxypropylmethylcellulose | 500 g |
| Lactose | 6.87 kg |
| Low-substitution hydroxypropylcellulose | 1.5 kg |
| Magnesium stearate | 30 g |

Lactose and low-substitution hydroxypropylcellulose are added to a suspension of nilvadipine and hydroxypropylmethylcellulose in anhydrous ethanol (5 liter) and the resulting mixture is stirred. Then, the organic solvent is removed under reduced pressure to obtain a solid dispersion composition. This composition is formulated into fine granules in accordance with a conventional procedure and, after adding magnesium stearate, the resulting fine granules are formulated into tablets in accordance with a conventional procedure. Each tablet contains 2 mg of nilvadipine.

### Example 4

The respective tablets obtained in Example 3 are film-coated with a coating layer made of hydroxypropylmethylcellulose (5.1 mg), titanium dioxide (1.6 mg), polyethylene glycol 6000 (0.8 mg), talc (0.4 mg) and yellow iron oxide (0.1 mg) in accordance with a conventional procedure to obtain film-coated tablets each containing 2 mg of nilvadipine.

### INDUSTRIAL APPLICABILITY

As described above, the drug containing a dihydropyridine compound (I) represented by nilvadipine of the present invention has a therapeutic effect on cancer-associated neuropathy and AZOOR or related diseases thereof and, therefore, it is expected to exert an effective therapeutic effect. Also it is superior in safety.

Although the therapeutic effect on cancer-associated neuropathy and AZOOR or related diseases thereof were described in the specification of the present invention, it is presumed from the results of the above pharmacological study of the dihydropyridine compound (I) that not only the therapeutic effect on cancer-associated neuropathy and AZOOR or related diseases thereof, but also the therapeutic effect on other opthalmic diseases are exerted. For example, other opthalmic diseases are following diseases.

### Classification of Eye Diseases:

### Retinal, vitreous diseases

1) diabetic retinopathy
2) retinal artery occlusion
3) retinal vein occlusion
4) central serous chorioretinopathy
5) macular degeneration
6) retinal detachment
7) retinal pigmentary degeneration
8) macular neovascularization
9) macular hole
10) proliferative vitreoretinopathy

### Glaucoma

1) primary open-angle glaucoma
2) primary angle-closure glaucoma
3) normal intraocular pressure glaucoma
4) neovascular glaucoma

### Vision disorders

1) amblyopia
2) night blindness

Fig. 1 shows the effect on apoptosis caused by CAR.
Fig. 2 shows the result of an examination of static visual field within the central 30 degrees using Humphrey visual field analyzer on November 10, 1999 (right eye).
Fig. 3 shows the result of an examination of static visual field within the central 30 degrees using Humphrey visual field analyzer on November 10, 1999 (left eye).
Fig. 4 shows the result of an examination of static visual field within the central 30 degrees using Humphrey visual field analyzer on January 25, 2000 (right eye).
Fig. 5 shows the result of an examination of static visual field within the central 30 degrees using Humphrey visual field analyzer on January 25, 2000 (left eye).

## Claims

1. A therapeutic agent for cancer-associated neuropathy and AZOOR or related diseases thereof, which contains a calcium antagonist as an active ingredient.

2. The therapeutic agent for cancer-associated neuropathy and AZOOR or related diseases thereof according to claim 1, wherein the calcium antagonist is a dihydropyridine-based calcium antagonist, a piperazine-based calcium antagonist, a phenylalkylamine-based calcium antagonist, a benzothiazepin-based calcium antagonist or a myosin light chain kinase (MLCK) inhibitory calcium antagonist.

3. The therapeutic agent for cancer-associated neuropathy and AZOOR or related diseases thereof according to claim 2, wherein the calcium antagonist is a dihydropyridine compound represented by the general formula (I): 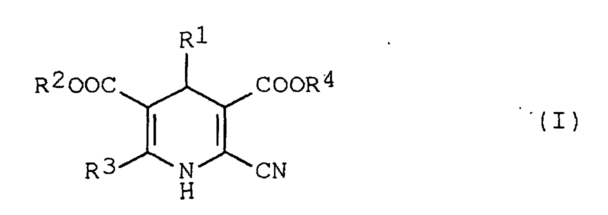 wherein R¹ represents nitrophenyl, and R², R³ and R⁴ each represents a lower alkyl group, or a salt thereof.

4. The therapeutic agent for cancer-associated neuropathy and AZOOR or related diseases thereof according to claim 3, wherein the dihydropyridine-based calcium antagonist is nilvadipine.

5. The therapeutic agent for cancer-associated neuropathy according to claim 4, wherein the cancer-associated neuropathy is cancer-associated retinopathy or melanoma-associated retinopathy.

6. The therapeutic agent for AZOOR or related diseases thereof according to claim 4, wherein AZOOR or related diseases thereof are AZOOR, MEWDS, AIBSES, AMN or P-POHS.

7. A method for treating cancer-associated neuropathy and AZOOR or related diseases thereof, which comprises administering a calcium antagonist to human or animal.

8. The method for treating cancer-associated neuropathy and AZOOR or related diseases thereof according to claim 7, wherein the calcium antagonist is a dihydropyridine-based calcium antagonist, a piperazine-based calcium antagonist, a phenylalkylamine-based calcium antagonist, a benzothiazepin-based calcium antagonist or a myosin light chain kinase (MLCK) inhibitory calcium antagonist.

9. The method for treating cancer-associated neuropathy and AZOOR or related diseases thereof according to claim 8, wherein the calcium antagonist is a dihydropyridine compound represented by the general formula (I): 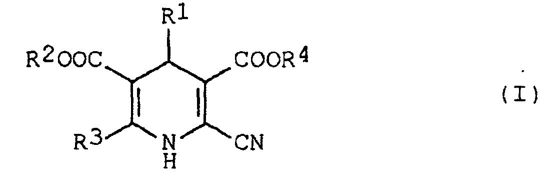 wherein R¹ represents nitrophenyl, and R², R³ and R⁴ each represents a lower alkyl group, or a salt thereof.

10. The method for treating cancer-associated neuropathy and AZOOR or related diseases thereof according to claim 9, wherein the dihydropyridine-based calcium antagonist is nilvadipine.

11. The method for treating cancer-associated neuropathy according to claim 10, wherein the cancer-associated neuropathy is cancer-associated retinopathy or melanoma-associated retinopathy.

12. The method for treating AZOOR or related diseases thereof according to claim 10, wherein AZOOR or related diseases thereof are AZOOR, MEWDS, AIBSES, AMN or P-POHS.

13. Use of a calcium antagonist for manufacturing of a pharmaceutical agent for treatment of cancer-associated neuropathy and AZOOR or related diseases thereof.

14. Use of the calcium antagonist according to claim 13, wherein the calcium antagonist is a dihydropyridine-based calcium antagonist, a piperazine-based calcium antagonist, a phenylalkylamine-based calcium antagonist, a benzothiazepin-based calcium antagonist or a myosin light chain kinase (MLCK) inhibitory calcium antagonist.

15. Use of the calcium antagonist according to claim 14, wherein the calcium antagonist is a dihydropyridine compound represented by the general formula (I): 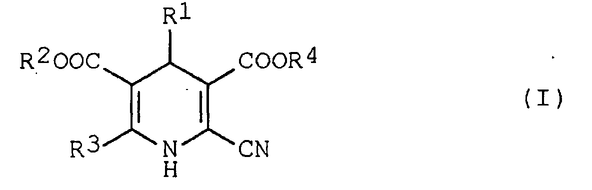 wherein R¹ represents nitrophenyl, and R², R³ and R⁴ each represents a lower alkyl group, or a salt thereof.

16. Use of the calcium antagonist according to claim 15, wherein the dihydropyridine-based calcium antagonist is nilvadipine.

17. Use of the calcium antagonist according to claim 16, wherein the cancer-associated neuropathy is cancer-associated retinopathy or melanoma-associated retinopathy.

18. Use of the calcium antagonist according to claim 16, wherein AZOOR or related diseases thereof is AZOOR, MEWDS, AIBSES, AMN or P-POHS.
